# EUROPÄISCHE PATENTSCHRIFT

(11) **EP 0 764 463 B1**
(45) Veröffentlichungstag und Bekanntmachung des Hinweises auf die Patenterteilung: **23.04.2003**
(21) Anmeldenummer: 96114801.2
(22) Anmeldetag: 16.09.1996
(51) Int. Cl.: B01J 23/58, C07C 5/08

(54) **Palladium-haltiger Trägerkatalysator zur selektiven katalytischen Hydrierung von Acetylen in Kohlenwasserstoffströmen**
Palladium containing supported catalyst for the selective hydrogenation of acetylen in hydrocarbon streams
Catalyseur supporté contenant du palladium pour l'hydrogénation sélective de l'acétylène dans un courant d'hydrocarbures

(30) Priorität: 23.09.1995 DE 19535402
(43) Veröffentlichungstag der Anmeldung: 26.03.1997
(73) Patentinhaber: BASF AKTIENGESELLSCHAFT, 67056 Ludwigshafen (DE)
(72) Erfinder: Flick, Klemens, Dr., 76863 Herxheim (DE); Herion, Christof, Dr., 68526 Ladenburg (DE); Allmann, Hans-Martin, 74867 Neunkirchen (DE)

(56) Entgegenhaltungen:
- EP-A- 0 723 811
- DE-A- 3 312 252
- DE-A- 3 508 371
- FR-A- 1 255 966
- US-A- 3 325 556
- DATABASE WPI Section Ch, Week 8448 Derwent Publications Ltd., London, GB; Class A41, AN 84-296981 XP002071109 & JP 59 182 890 A (SUMITOMO CHEM CO LTD)

## Beschreibung

Die vorliegende Erfindung betrifft neue Trägerkatalysatoren auf Basis von Siliciumdioxid, die zur selektiven katalytischen Hydrierung von Acetylen in Kohlenwasserstoffströmen geeignet sind.

Weiterhin betrifft die Erfindung ein Verfahren zur Herstellung dieser Katalysatoren sowie ein Verfahren zur selektiven Hydrierung von Acetylen in Kohlenwasserstoffströmen unter Verwendung der erfindungsgemäßen Katalysatoren.

Acetylen ist wegen seiner Neigung zur Polymerisation und zur Inaktivierung von Übergangsmetallkatalysatoren für verschiedene technische Reaktionen ein unerwünschter Bestandteil von Einsatzstoffen. Insbesondere ist Acetylen in den sogenannten C₂-Strömen aus Steamcrackern technisch nachteilig. Diese Ströme enthalten im wesentlichen Ethylen und Ethan sowie geringe Mengen an Acetylen. Für einen Einsatz dieser Ströme zur Herstellung von Polyethylen muß der Acetylengehalt auf weniger als 1 ppm abgesenkt werden. An für eine Hydrierung von Acetylen geeignete Katalysatoren werden hohe Anforderungen hinsichtlich ihrer Selektivität und Aktivität gestellt, denn die Hydrierung soll ohne Verlust an Ethylen erfolgen.

Überwiegend werden zu diesem Zweck Palladium-haltige Trägerkatalysatoren auf Basis von Aluminiumoxid eingesetzt. So sind Katalysatoren vorgeschlagen worden, die Palladium auf makroporösen Al₂O₃-Trägern mit einer BET-Oberfläche von 0,1 bis 2 m²/g enthalten (JP-B 80/047 015; Chem. Abstr., Vol. 82, 169991), die Palladium und wahlweise zusätzlich Chrom auf Trägern mit einer Oberfläche von weniger als 5 m²/g tragen (US-A 4 577 047), die neben Palladium Silber (EP-A 64 301), Gold (EP-A 89 252), Lithium (US-A 3 325 556), Chrom (DE-A 12 84 403) oder Kalium als Promotoren tragen (Park et al., J. Chem. Soc., Chem. Commun. 1991, 1188; Ind. Eng. Chem Res. 31 (1992) 469).

TiO₂ als weiteres Trägermaterial für Palladiumkatalysatoren für die selektive Acetylenhydrierung ist in der US-A 4 839 329 beschrieben. Palladium-Blei-Katalysatoren auf Basis von CaCO₃ lehrt die US-A 4 906 800. Die DE-A 21 56 544 betrifft Palladium-Zink-Katalysatoren auf Siliciumdioxid.

Die deutsche Patentanmeldung P 19 500 366.7 betrifft PalladiumTrägerkatalysatoren, die durch Tränkung eines Trägers mit einem Palladiumsol hergestellt werden.

DE-A-3 312 252 lehrt Hydrierkatalysatoren, die neben 0,1 - 60 Gew.% Palladium auch 0,1 - 10 Gew.-% Kaliumoxid auf einem inerten Träger enthalten, und die durch Tränkung einer Katalysatorvorstufe, die bereits das Palladium und den Träger enthält, mit einer Lösung einer Kaliumverbindung hergestellt werden. Weiterhin lehrt diese Schrift die Verwendung solcher Katalysatoren zur Hydrierung von acetylenischen Kohlenwasserstoffen.

US-A-3,325,556 offenbart ein Verfahren zur Hydrierung von Acetylen in C2-Strömen, bei dem ein edelmetallhaltiger Katalysator mit einem lithiumhaltigen Aluminiumoxidträger verwendet wird.

Zur Hydrierung geringer Mengen Acetylen in Kohlenwasserstoffströmen ist es bei Verwendung aller genannter Katalysatoren erforderlich, dem zu hydrierenden Strom Kohlenmonoxid beizumischen, um die Selektivität des jeweiligen Katalysators zu erhöhen. Diese Verfahrensweise hat den Nachteil, daß die selektivitätssteigerndde Wirkung des Kohlenmonoxids stark temperaturabhängig ist. Große Temperaturgradienten im Katalysatorbett haben daher eine Verschlechterung der Selektivität zur Folge. Außerdem erfordert die Dosierung relativ kleiner Mengen Kohlenmonoxid in vergleichsweise große Ströme einen hohen Meß- und Regelaufwand.

Weiterhin enthalten die genannten Katalysatoren zum Teil große Mengen teuerer Edelmetallen, die ihrer großtechnischen Anwendung aus wirtschaftlichen Gründen entgegenstehen.

Es bestand daher die Aufgabe, Katalysatoren bereitzustellen, die diese Nachteile nicht aufweisen. Insbesondere sollen sie in der Lage sein, auch geringe Mengen an Acetylen in Kohlenwasserstoffströmen mit hoher Selektivität ohne Zusatz von Kohlenmonoxid zu hydrieren.

Demgemäß wurden Trägerkatalysatoren auf Basis von Siliciumdioxid mit einem Palladiumgehalt von 0,001 bis 1 Gew.-% und mindestens einem Promotormetall aus den Gruppen 1 und 2 des Periodischen Systems der Elemente in einer Menge von 0,005 bis 5 Gew.-%, jeweils bezogen auf den Trägerkatalysator, gefunden, die durch Tränken eines Siliciumdioxidträgers mit einer mindestens ein Promotormetall enthaltenden Lösung, Trocknen des so erhaltenen getränkten Trägers, Tränken mit einer Palladium enthaltenden Lösung, Trocknen und Calcinieren erhältlich sind.

Weiterhin wurde ein Verfahren zur Herstellung der erfindungsgemäßen Katalysatoren sowie ein Verfahren zur Hydrierung von Acetylen unter Verwendung der Katalysatoren gefunden.

Die erfindungsgemäßen Trägerkatalysatoren werden durch schrittweise Tränkung eines Siliciumdioxidträgers hergestellt. Diese Träger können in beliebiger Form eingesetzt werden, beispielsweise als Splitt, Granulat oder Tabletten, bevorzugt aber als Stränge. Es hat sich als vorteilhaft erwiesen, Siliciumdioxid mit einer BET-Oberfläche von mindestens 50 m²/g zu verwenden, bevorzugt werden Träger mit 100 bis 300 m²/g Oberfläche. Die chemische Herstellung des verwendeten Siliciumdioxids spielt keine erkennbare Rolle für die Leistungsfähigkeit der erfindungsgemäßen Katalysatoren, beispielsweise kann gefällte oder pyrogene Kieselsäure verwendet werden.

In der ersten Tränkung wird der Promotor auf den Träger aufgebracht. Vorteilhaft versetzt man dazu den Träger mit einer solchen Menge an Lösung, die der Träger maximal adsorbieren kann. In dieser Lösung befindet sich eine solche Menge an Promotormetallen, daß der abschließend calcinierte Katalysator eine Menge von 0,005 bis 5 Gew.-% Promotormetall enthält. Die geeigneten Mengenverhältnisse kann der Fachmann leicht aufgrund weniger Vorversuche ermitteln.

Als Promotormetalle kommen Alkali- und Erdalkalimetalle in Betracht wie Lithium, Natrium, Kalium, Rubidium, Caesium, Calcium, Strontium und Barium. Bevorzugt hiervon sind Rubidium, Strontium und Barium, ganz besonders aber Kalium. Es kann sowohl eines wie auch mehrere der genannten Promotorenmetalle auf den Träger gebracht werden. Die Promotorenmetalle werden üblicherweise in Form ihrer Salze in Lösungsmitteln, bevorzugt in Wasser, gelöst. Als Salze sind vor allem solche Salze zu nennen, die sich in der Calcinierung leicht in die entsprechenden Oxide überführen lassen, beispielsweise Hydroxide, Carbonate, Nitrate, Acetate und Formiate der genannten Promotormetalle, von denen die Hydroxide bevorzugt sind.

Nach erfolgter Tränkung mit den Promotormetallen wird der so erhaltene getränkte Träger getrocknet. Die Trocknung erfolgt in der Regel bei Temperaturen unter 300°C, da es bei längerer Einwirkung höherer Temperaturen in vermehrtem Maße zur Bildung von Silicaten kommt. Die Trocknung dauert im allgemeinen 5 bis 20 h. Sie ist beendet, wenn keine erkennbaren Mengen an Lösungsmittel mehr freigesetzt werden.

In einer zweiten Tränkung wird Palladium in an sich bekannter Weise auf den vorbehandelten Träger aufgebracht. Dies geschieht wie im Fall der Promotormetalle vorzugsweise durch Tränkung mit einer Lösungsmittelmenge, die vollständig vom Träger aufgenommen werden kann. Der Träger wird mit einer Palladiumsalzlösung versetzt. Als Lösungsmittel für das Palladiumsalz kommt bevorzugt Wasser in Betracht. Es werden nur echte Lösungen verwendet, d.h. Palladium enthaltende Sole werden nicht eingesetzt. Als Palladiumsalze sind beispielhaft Palladiumnitrat, -acetat, -acetylacetonat und -chlorid zu nennen. Einige dieser Palladiumsalzlösungen sind bedingt durch die verwendeten Anionen sauer. Neutral reagierende Lösungen werden in einer bevorzugten Ausführungsform vor der Tränkung durch Zugabe von beispielsweise Mineralsäuren sauer gestellt.

Nach der Tränkung des Trägers mit der Palladiumsalzlösung wird dieser getrocknet. Die Trocknungstemperatur beträgt im allgemeinen 100 bis 200°C über einen Zeitraum von 5 bis 20 h. Der Träger wird dabei bevorzugt bewegt, um eine gleichmäßige Trocknung zu erzielen.

Anschließend werden die getrockneten Träger calciniert, was in der Regel bei Temperaturen von 300 bis 700°C, vorzugsweise von 320 bis 450°C über einen Zeitraum von 0,5 bis 8 h erfolgt.

Vor dem Einsatz der erfindungsgemäßen Katalysatoren in einem Verfahren zur Hydrierung von Acetylen können diese durch Reduktion mit Wasserstoff oder einem Wasserstoff enthaltenden Gas bei Temperaturen, die im allgemeinen bei 100 bis 550°C liegen, aktiviert werden, wobei der Wasserstoffpartialdruck zweckmäßigerweise 1 bis 300 bar beträgt und die Reduktion solange ausgeführt wird, bis sich kein Wasser mehr bildet.

Die erfindungsgemäßen Katalysatoren weisen eine dünne Schicht von Palladium auf einem Promotormetalloxid auf. Durch diesen Aufbau des Katalysators wird eine hohe Selektivität erzielt.

Die erfindungsgemäßen Katalysatoren können zum Beispiel zur selektiven Hydrierung von Acetylen in Kohlenwasserstoffströmen eingesetzt werden. Von besonderem Interesse sind dabei solche Ströme aus Steamcrackern, die neben den Hauptbestandteilen Ethylen und Ethan Acetylen in Mengen von 0,01 bis 5 Vol.-% enthalten.

Acetylen enthaltende Kohlenwasserstoffströme werden in der Gasphase bei Wasserstoffdrücken, die in der Regel 10 bis 30 bar betragen, hydriert. Je nach der zu hydrierenden Acetylenmenge kann die Hydrierung einstufig oder mehrstufig erfolgen, wobei in an sich bekannter Weise Zwischenkühlungen und Wasserstoffeinspeisungen in jeden einzelnen Reaktor angewendet werden können. Eine adiabatische Fahrweise ist dabei bevorzugt und läßt sich für Acetylengehalte von unter 1 Vol-% leicht realisieren. Die Eintrittstemperatur der Acetylen enthaltenden Kohlenwasserstoffströme in den ersten Reaktor betragen im allgemeinen 15 bis 120°C, bevorzugt 25 bis 95°C. Bei Verwendung nur eines Reaktors liegt das molare Verhältnis von Wasserstoff zu Acetylen in der Regel bei 1,1 bis 2:1, bevorzugt bei 1,2 bis 1,6:1. Bei Verwendung mehrerer in Reihe geschalteter Reaktoren mit Wasserstoffzufuhr in jeden Reaktor kann es dagegen 0,6 bis 1,2:1 betragen.
Die erfindungsgemäßen Katalysatoren sind in der Hydrierung von Acetylen sehr aktiv und können bei relativ niedrigen Temperaturen eingesetzt werden. Sie sind auch bei geringen Acetylengehalten hochselektiv, ohne daß Kohlenmonoxid in der Hydrierstufe zudosiert werden muß. Es lassen sich Restacetylengehalte von ca. 1 ppm erzielen, wobei im Vergleich zu bekannten Katalysatoren mit geringen stöchiometrischen Wasserstoffüberschüssen gearbeitet werden kann.

### Beispiele

### Beispiel 1

### Herstellung eines Kalium-dotierten Katalysators

111 kg eines Kieselgelträgers (Durchmesser 4mm) mit einer BET-Oberfläche von 270 m²/g wurden mit 92 l einer Lösung von 330 g Kaliumhydroxid in Wasser getränkt. Der Träger wurde 16 h bei 120°C bewegt und getrocknet. Die zweite Tränkung wurde mit 95 l einer wäßrigen Lösung von Palladiumnitrat mit einem Palladiumgehalt von 33 g durchgeführt. Nach einer Trocknung bei 120°C über einen Zeitraum von 16 h wurden die Stränge bei 400°C 4 h calciniert.

### Beispiel 2

### Selektive Hydrierung von Acetylen mit einem erfindungsgemäßen Katalysator

In einem Rohrreaktor mit einem Durchmesser von 305 mm wurden 50 1 Katalysator gemäß Beispiel 1 gegeben. Der Reaktor wurde mit Stickstoff gespült. Der Katalysator wurde dann 3 h bei 150°C bei 20 bar mit Wasserstoff reduziert.

Der Reaktor wurde mit 150 m³/h eines Stromes, der neben 69,6 Vol.-% Ethylen und 29,6 Vol.-% Ethan 0,71 Vol.-% Acetylen enthielt, sowie mit Wasserstoff mit einer Eingangstemperatur von 35°C beschickt. Das molare Verhältnis von Wasserstoff zu Acetylen wurde dabei schrittweise reduziert. Tabelle 1 sind Einzelheiten zur Umsetzung zu entnehmen.

**Tabelle 1**

| mol H₂/mol Acetylen | Acetylengehalt nach Hydrierung (ppm) | Umsatz (%) | Selektivität (%) |
|---|---|---|---|
| 1,81 | < 0,1 | > 99,999 | 19 |
| 1,54 | < 0,1 | > 99,999 | 46 |
| 1,43 | < 0,1 | > 99,999 | 56 |

## Patentansprüche

1. Trägerkatalysator auf Basis von Siliciumdioxid mit einem Palladiumgehalt von 0,001 bis 1 Gew.-% und mindestens einem Promotormetall aus den Gruppen 1 und 2 des Periodischen Systems der Elemente in einer Menge von 0,005 bis 5 Gew.-%, jeweils bezogen auf den Trägerkatalysator, erhältlich durch Tränken eines Siliciumdioxidträgers mit einer mindestens ein Promotormetall enthaltenden Lösung, Trocknen des so erhaltenen getränkten Trägers, Tränken mit einer Palladium enthaltenden Lösung, Trocknen und Calcinieren.

2. Trägerkatalysator nach Anspruch 1, der Kalium, Rubidium, Strontium oder Barium als Promotormetall enthält.

3. Trägerkatalysator nach Anspruch 1 oder 2, der durch Tränken eines Siliciumdioxidträgers mit Kalilauge, Trocknen und anschließende Tränkung mit einer Palladiumnitrat enthaltenden Lösung hergestellt wird.

4. Trägerkatalysator nach den Ansprüchen 1 bis 3, der durch Tränken eines Siliciumdioxidträgers mit einer BET-Oberfläche von 100 bis 300 m²/g hergestellt wird.

5. Verfahren zur Herstellung eines Trägerkatalysators mit einem Palladiumgehalt von 0,001 bis 1 Gew.-% und mindestens einem Promotormetall aus den Gruppen 1 und 2 des Periodischen Systems der Elemente in einer Menge von 0,005 bis 5 Gew.-%, jeweils bezogen auf den Trägerkatalysator, **dadurch gekennzeichnet, daß** man einen Siliciumdioxidträger mit einer mindestens ein Promotormetall enthaltenden Lösung tränkt, den so erhaltenen getränkten Träger trocknet, mit einer ein Palladiumsalz enthaltenden Lösung tränkt, trocknet und calciniert.

6. Verwendung eines Katalysators gemäß den Ansprüchen 1 bis 4 zur katalytischen Hydrierung von Acetylen in Kohlenwasserstoffströmen.

7. Verwendung nach Anspruch 6, **dadurch gekennzeichnet, daß** man Acetylen in Gegenwart von Ethylen hydriert.

8. Verfahren zur selektiven katalytischen Hydrierung von Acetylen mit Wasserstoff in Kohlenwasserstoffströmen, **dadurch gekennzeichnet, daß** man die Hydrierung in der Gasphase an einem Katalysator gemäß den Ansprüchen 1 bis 4 vornimmt.

9. Verfahren nach Anspruch 8, **dadurch gekennzeichnet,daß** man Acetylen in einem im wesentlichen Ethylen und Ethan enthaltenden Strom selektiv hydriert, der 0,01 bis 5 Vol.-% Acetylen enthält.

## Claims

1. A silica-supported catalyst comprising from 0.001 to 1 % by weight, based on the supported catalyst, of palladium and from 0.005 to 5 % by weight, based on the supported catalyst, of at least one promoter metal of groups 1 and 2 of the periodic table, obtainable by impregnating a silica support with a solution comprising at least one promoter metal, drying the impregnated support, impregnating with a palladium-containing solution, drying and calcining.

2. A supported catalyst as claimed in claim 1, including potassium, rubidium, strontium or barium as promoter metal.

3. A supported catalyst as claimed in claim 1 or 2, obtainable by impregnating a silica support with aqueous potassium hydroxide solution, drying and then impregnating with a solution comprising palladium nitrate.

4. A supported catalyst as claimed in any of claims 1 to 3, obtainable by impregnating a silica support having a BET' surface area of from 100 to 300 m²/g.

5. A process for preparing a supported catalyst comprising from 0.001 to 1 % by weight, based on the supported catalyst, of palladium and from 0.005 to 5 % by weight, based on the supported catalyst, of at least one promoter metal of groups 1 and 2 of the periodic table, which comprises impregnating a silica support with a solution comprising at least one promoter metal, drying the impregnated support, impregnating with a palladium-containing solution, drying and calcining.

6. The use of a catalyst as claimed in any of claims 1 to 4 for the catalytic hydrogenation of acetylene in hydrocarbonaceous streams.

7. A use as claimed in claim 6, whereby acetylene is hydrogenated in the presence of ethylene.

8. A process for the selective catalytic hydrogenation of acetylene with hydrogen in hydrocarbonaceous streams, which comprises hydrogenating in the gas phase over a catalyst as claimed in any of claims 1 to 4.

9. A process as claimed in claim 8, wherein acetylene is selectively hydrogenated in an essentially ethylene- and ethane-containing stream that includes from 0.01 to 5 % by volume of acetylene.

## Revendications

1. Catalyseur déposé sur un support, à base de dioxyde de silicium, possédant une teneur en palladium de 0,001 à 1 % en poids et comprenant au moins un métal promoteur choisi parmi les groupes 1 et 2 du système périodique des éléments en une quantité de 0,005 à 5 % en poids, chaque fois rapportée au catalyseur déposé sur un support, que l'on obtient par imprégnation d'un support à base de dioxyde de silicium avec une solution contenant au moins un métal promoteur, par séchage du support imprégné ainsi obtenu, par imprégnation avec une solution contenant du palladium, par séchage et par calcination.

2. Catalyseur déposé sur un support selon la revendication 1, qui contient du potassium, du rubidium, du strontium ou du baryum à titre de métal promoteur.

3. Catalyseur déposé sur un support selon la revendication 1 ou 2, que l'on prépare par imprégnation d'un support à base de dioxyde de silicium avec de la potasse caustique, par séchage et par imprégnation ultérieure avec une solution contenant du nitrate de palladium.

4. Catalyseur déposé sur un support selon l'une quelconque des revendications 1 à 3, que l'on prépare par imprégnation d'un support à base de dioxyde de silicium possédant une surface selon BET de 100 à 300 m²/g.

5. Procédé pour la préparation d'un catalyseur déposé sur un support possédant une teneur en palladium de 0,001 à 1 % en poids et comprenant au moins un métal promoteur choisi parmi les groupes 1 et 2 du système périodique des éléments en une quantité de 0,005 à 5 % en poids, chaque fois rapportée au catalyseur déposé sur un support, **caractérisé en ce qu'**on imprègne un support à base de dioxyde de silicium avec une solution contenant au moins un métal promoteur, on sèche le support imprégné ainsi obtenu, on l'imprègne avec une solution contenant un sel de palladium, on le sèche et on le calcine.

6. Utilisation d'un catalyseur selon les revendications 1 à 4, pour l'hydrogénation catalytique d'acétylène dans des courants d'hydrocarbures.

7. Utilisation selon la revendication 6, **caractérisé en ce qu'**on soumet de l'acétylène à une hydrogénation en présence d'éthylène.

8. Procédé pour l'hydrogénation catalytique sélective d'acétylène avec de l'hydrogène dans des courants d'hydrocarbures, **caractérisé en ce qu'**on procède à l'hydrogénation en phase gazeuse sur un catalyseur selon les revendications 1 à 4.

9. Procédé selon la revendication 8, **caractérisé en ce qu'**on soumet de l'acétylène à une hydrogénation sélective dans un courant contenant essentiellement de l'éthylène et de l'éthane, qui contient de l'acétylène à concurrence de 0,01 à 5 % en volume.
